(19) Europäisches Patentamt
European Patent Office
Office européen des brevets

(11) Publication number: 0 366 632 A1

(12) # EUROPEAN PATENT APPLICATION

(21) Application number: 89850291.9

(22) Date of filing: 06.09.89

(51) Int. Cl.[5]: C07C 59/185 , C07C 59/195 , C07C 69/716 , C07C 69/72 , A61K 31/19 , A61K 31/22

A request for correction of typographic errors in the description and claims has been filed pursuant to Rule 88 EPC. A decision on the request will be taken during the proceedings before the Examining Division (Guidelines for Examination in the EPO, A-V, 2.2).

(30) Priority: 07.09.88 SE 8803143

(43) Date of publication of application: 02.05.90 Bulletin 90/18

(84) Designated Contracting States: ES GR

(71) Applicant: KABIVITRUM AB

S-112 87 Stockholm(SE)

(72) Inventor: Lindgren, Svante
Lättsta Skepptuna
S-195 00 Märsta(SE)
Inventor: Granelli, Kristina
Väderkvarnsgatan 8 B
S-753 29 Uppsala(SE)
Inventor: Campos, Kerstin
Götavägen 75
S-183 67 Täby(SE)
Inventor: Grimfors, Christer
Brobyvägen 54
S-183 40 Täby(SE)
Inventor: Johansson, Mats
Köpenhamnsgatan 24
S-164 42 Kista(SE)
Inventor: Stenström, Thomas
C/O Sandberg, Skanegatan 92
S-116 37 Stockholm(SE)
Inventor: Jönsson, Ake
Fotbollsvägen 6
S-151 59 Södertälje(SE)
Inventor: Werner, Tom
Nysätravägen 5
S-131 33 Nacka(SE)

(74) Representative: Onn, Thorsten et al
AB Stockholms Patentbyra Zacco & Bruhn
P.O. Box 3129
S-103 62 Stockholm(SE)

(54) **Alternative energy substrates.**

(57) Compounds of the general formula

A - B

where A is a carboxylic acid group having a carbon chain of 3-10 carbon atoms and preferably being branched, and which includes one or more oxo groups, and B is hydrogen, a pharmaceutically acceptable metal atom, an alcohol bound as ester and having 1-5 carbon atoms and 1-3 hydroxy groups, or a glycerol group bound as ester, can be used as a nutrient substrate, an antimicrobial and antiviral agent, and/or as an agent for influencing the central nervous system.

The invention also relates to compositions intended for the aforementioned usages and containing at least one compound A - B.

EP 0 366 632 A1

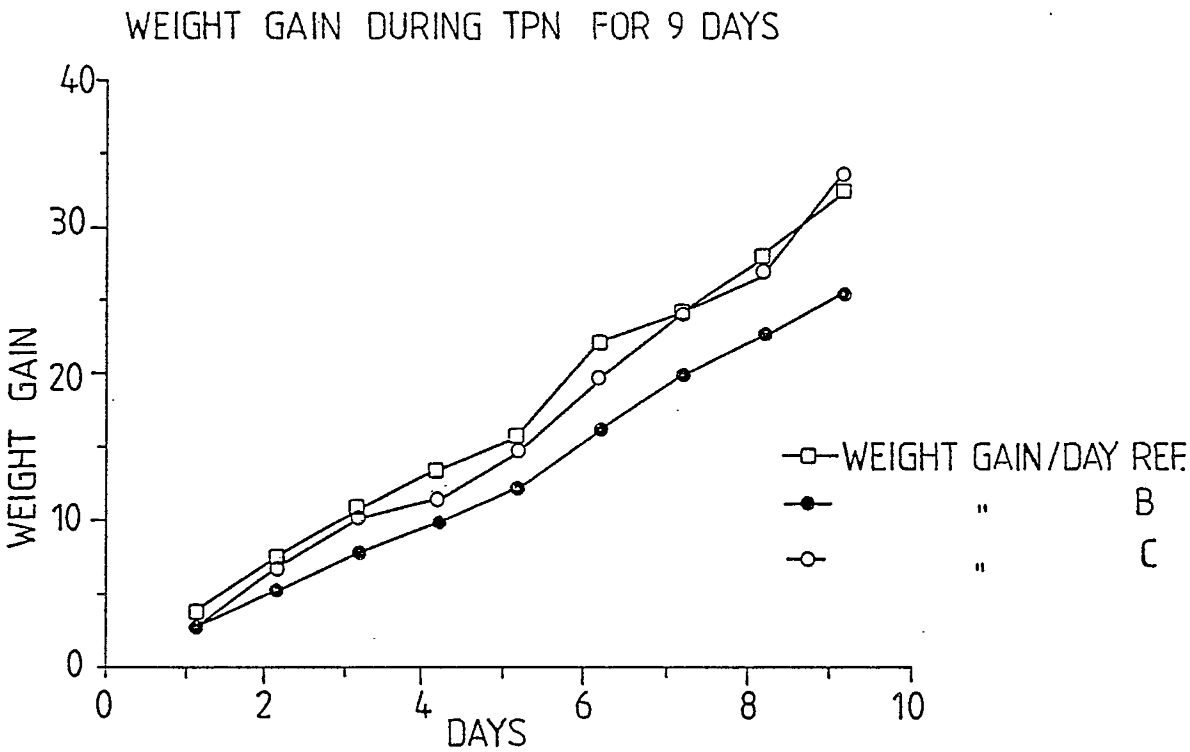
WEIGHT GAIN DURING TPN FOR 9 DAYS
40
30
20
10
0
WEIGHT GAIN
0
2
4
6
8
10
DAYS
WEIGHT GAIN/DAY REF.
" B
" C

FIG.1

## Alternative energy substrate

The present invention relates to a pharmaceutical substrate and pertains to the field of clinical nutrition. This field is concerned with all forms of enteral and parenteral nutrition of mammals.

Those groups of patients subjected to various forms of clinical nutrition often have a poor nutrition status. This status may be the cause of serious trauma, such as deep and extensive burns, surgical trauma, serious body injuries, various forms of cancer or sepsis. Other groups comprise patients who are unable to eat, for physiological, anatomical or other reasons, for instance unconciousness. In the case of serious trauma, however, the metabolic picture is complicated, because of the poor utilization of glucose in the peripheral tissues. Although the glucose and insuline levels of the blood increase, no energy, or relatively little energy is gained thereby. A so-called peripheral glucose intolerance with insulin resistance has arisen (Gump F.E., Long C., Killian P and Kinney J., M.,; J. TRAUMA 14(5): 378-388, 1974. / Black P.R., Brooks D.C., Bessey P.Q., Wolfe R.R. and Wilmoore D.W. ANN.SURG. 196(4); 420-435, 1982 / Drobney E.C., Abramson E.C. and Baumann G.J.CLIN. ENDOCRINOL.METAB. 58(4):710, 1984 etc.).

In the case of glucose intolerance, the oxidation of fat is also reduced and the ketone body metabolism is unchanged, which means that the total energy recovery is impaired (Ryan et al METAB. 23(11), 1081 1974., / O,Donnel T.F., Blacburn G.L. "Proteolysis associated with a deficit of periferal energy fuel substrates in septic man. SURGERY 80; 192-200, 1972). In a situation such as this, the body mobilizes different body proteins as an energy source, which results in a more or less extreme protein catabolic situation.

Many attempts have been made to normalize the clinical or pathological picture of the illness and restitute the concentration of branched amino acids in different stress situations, but with varying results.

As before mentioned, in trauma and sepsis conditions, there prevails a peripheral glucose intolerance with insulin resistance. This results in high plasma concentrations of glucose, lactate, pyruvate and alanine, and a radical change in muscle energy conversion.

The release of leucine, valine, isoleucine, asparagine, aspartic acid, glutamine and glutamic acid occurs in normal test subjects (5 $\mu mol/m^2$ and minute) but increases in the case of sepsis and various trauma by about 80% (8-9 $\mu mol/m^2$ and minute).

Plasma exhibits low concentrations of ketone bodies, branched amino acids and albumin, and also high concentrations of triglycerides, aromatic amino acids (tryptophan, phenylalanine and tyrosine), methionine and histidine, and increasing concentrations of circulating lactate and pyruvate. If this pathological picture is not normalized, the patient will die (Birhahn R.H. and Border J.R. Am. J. Clin. Nutr. 31., 436-441, 1978, / Birkhahn R.H. and Border J.R. JPEN 5(1), 1981.

The branched amino acids are oxidized substantially in the peripheral protein depots (the muscles), primarily leucine, although the remaining branched amino acids, isoleucine and valine are also oxidized. This means, for instance, that the pool of branched amino acids possessed by this group of patients will gradually be depleted. This will, in turn, result in a reduced pro tein synthesis in the liver.

The liver utilizes amino acids as an energy substrate to varying degrees, primarily alanine. Splanchnicus (walls of the intestines or the like) mainly utilizes glutamine as an energy source (Ryan N.T., Blackburn G.L. and Clowes Jr. G.H.A. METAB. 23(11), 1081, 1974. / O'Donnel Jr. T.F., Clowes Jr. G.H.A., Blackburn G.L., Long C.L. and Kinney J.M. SURGERY 68(1); 168-174, 1970.)

By way of summary, the following metabolic conditions exist in, for instance, sepsis and also trauma conditions:

a) Increased proteolysis of muscle proteins and increased oxidation of amino acids.

b) Hyperglycemia. (The lever continues to produce glucose despite high insulin levels).

c) Increased peripheral take-up of glucose.

d) Increased production of pyruvate.

e) Reduced oxidation of pyruvate, as a result of restraint in the pyruvate metabolism.

f) Increased production of lactate, alanine and pyruvate.

g) Decreased sensitivity to the effect of insuline on the glucose metabolism in the muscle cells (insulin resistance).

h) A decrease in the plasma concentration of glutamine is also present in many trauma situations.

A glucose intolerance with insulin resistance has been established as a postreceptoric defect (Black P.R., Brooks O.C., Bessey P.Q., Wolfe R.R. and Wilmore D.W. ANN. SURG. 196(4) 420-435 1982; / Drobny E.C., Abramson E.C. and Baum,ann G.J. CLIN. ENDOCR. METAB. 58(4) 710, 1984.) This is characterised in that glucose is meta bolized solely to and including pyruvate. This inhibition or restraint takes place either at the pyruvate dehydrogenase and/or the pyruvate carrier. This end result is that the mitocondria matrix

receives less energy substrate from the glycolysis for conversion to acetyl-CoA. Since the glucose requirement of the cell cannot be satisfied, the concentration of insulin again increases. The insulin levels increase, in this way, without the cell receiving more energy from glucose. This results in a diabetic-like situation.

A compilation of the above facts illustrates the complex metabolic conditions for the branched amino acids in stress situations. The net result is a disturbed energy conversion, in which only available substrates (BCAA, branched amino acids) are converted to energy, although only to limited extents.

Certain matters should be observed when administering total nutrition parenterally to patients of this category:

1) The administration of solely glucose, intravenously, in the case of glucose intolerance has only negative effects in the main.

2) The administration of fat, intravenously, in the case of, e.g., sepsis, can in certain cases be directly contraindicative. A liver insufficiency usually exists with a subsequent changed fat metabolism.

3) It is necessary to find a glucose substitute in the aforesaid stress situation with glucose intolerance.

4) The administration of branched amino acids gives a varying result and can, in certain instances, be stressful in itself. Furthermore, oxidation of these amino acids results in an increase in the concentrations of nitrogen metabolites in the blood, a circumstance which must be strongly questioned in view of the fact that in the case of sepsis with, for instance, an endo-toxine shock with impaired liver function, the renal secretion of e.g., ammonia is impaired due to the lowered blood pressure, which must be considered highly toxic to the brain.

5) A possible, new substrate should be capable of replacing amino acids, glucose and fat from an energy aspect, even under normal metabolic conditions.

Pyruvate can, to a certain extent, disturb the dehydrogenase for the branched alpha-keto acids, via a functional effect. Furthermore, it has been found (in vitro) that high pyruvate concentrations can inhibit the formation of glutamic acid from alpha-keto-glutamic acid in the transamination of branched amino acids. Support for this is found in the fact that circulating blood concentrations of glutamine are lowered greatly in trauma cases. (Clowes Jr. G.H.A., Randall H.T. and Chac J. JP'EN 4(2) 195-205; 1980. / Van Hinsberg V.W., Veerkamp J.H., Engelen P.J.M. and Ghjisen W.J. BIOCHEM. MED. 20, 115-124, 1978; / Buse M.G., Biggers J.F., Karen J.F. Karen H.F. and Buse J.F.; J of BIOL. CHEM. 247 8085-96, 1972). The pyruvate dehydrogenase and/or the pyruvate carrier can, however, be inhibited competitively, to some extent, by alpha-ketoleucine (to a lesser degree by alpha-ketoisoleucine and alpha-ketovaline), which can be proven in invitro experiments.

In the case of invitro experiments carried out on the cells of rat livers, however, it was found that the decarboxylation rate was stimulated 2-9 times by branched alpha-keto acids in comparison with corresponding branched amino acids. This is an advantage when branched keto acids are able to replace glucose in part. Experiments in which branched keto acids are used as an energy additive have not earlier been carried out. Instead, experiments with branched keto acids have been directed to the administration of nitrogen-free amino acid substitutes to, for instance, uremics and liver patients (German Patent No. 2335216, US Patent No. 4 100 161,
4 100 160, EP 0 227 545).

It has been found difficult in practice, however, to produce and store solutions which contain branched alpha-keto acids in free form, since such solutions have extremely poor stability. Acids bound to different carriers, such as metals, alkali metals and alkaline earth metals for instance, also have poor stability. One proposed solution to the problem of the poor stability and durability of the keto acids during storage, is to use a concentrated solution which has been sterile-filtered and frozen. In use, the keto-acid concentrate is thawed and diluted to the correct concentration. (German Patent Specification No. 2 335 216 and others). This method, however, is impractical and also dangerous from an antimicrobial aspect. One method of bypassing the instable alpha-keto acids, is to produce beta-keto acids or gamma-keto acids, which are much more stable, particularly when they are esterified or bound in the carboxyl group in some other manner. As a metabolic substrate, it is assumed that these acids and esters are able to take part in the following reactions (the example below illustrates the metabolism of 4-methyl-3-oxo-pentanoic acid ethyl ester):

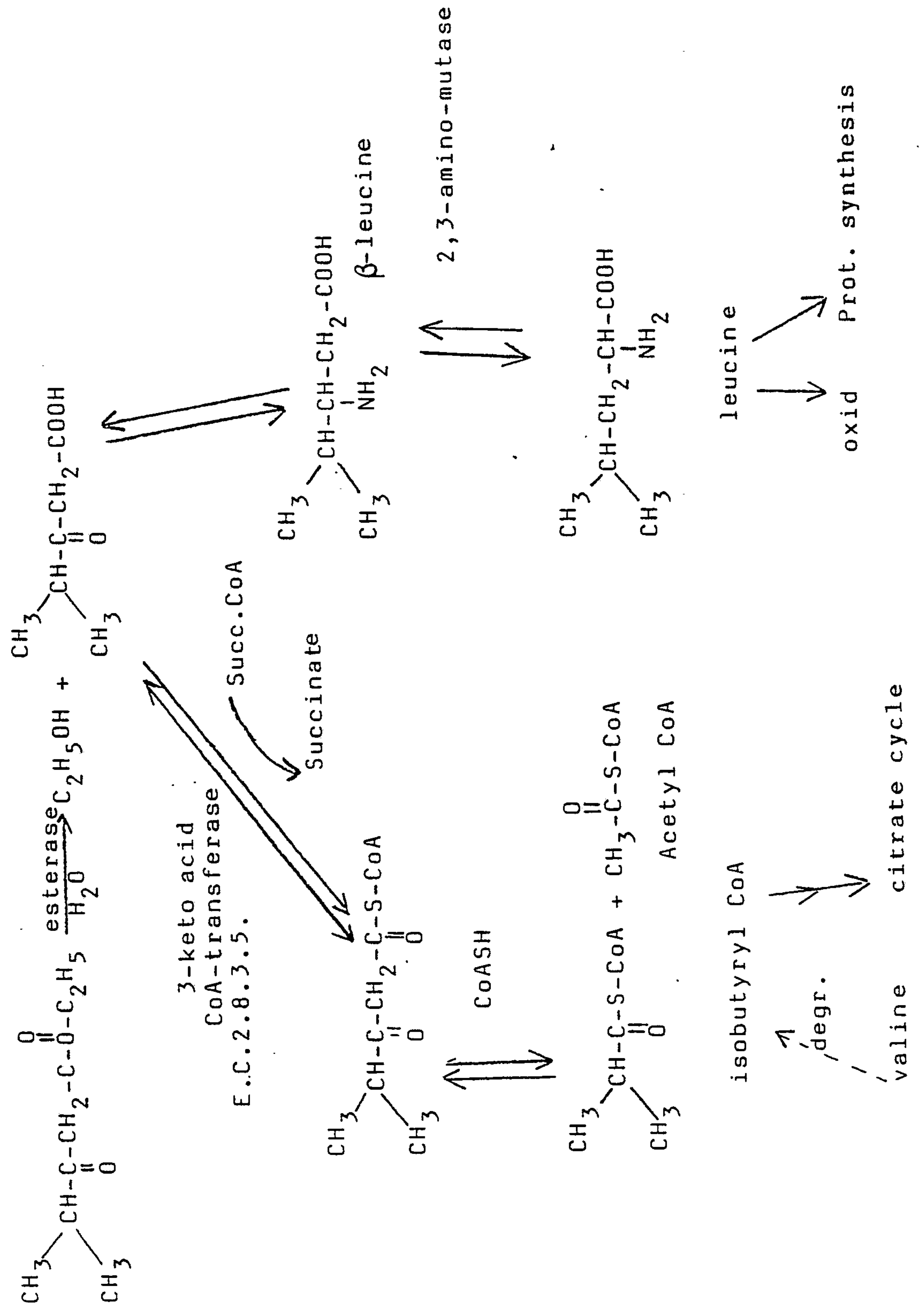


Poston J M: The relative carbon flux through the alpha-and beta-keto pathways of leucine metabolism. J Biol Chem 259(4): 2059-2061, 1984.

Poston J M: Cobalamin-dependent formation of leucine and beta-leucine by rat and human tissue. J Biol Chem 255(21)-:10057-10072, 1980).

The metabolism of, for instance, 4-methyl-3-oxo-pentanoic acid or beta-keto-isocaproate and beta-ketoisohexanoate (5-methyl-3-oxohexanoate) is described by the following authors:

Stern J R, Coon M J. del Campillo A: Enzymatic breakdown and symthesis of acetoacetate. Nature 171:28-30, 1953. Special article in Science 97(2526):490-492, 1943.
Stern J R, Coon M J, del Campillo A and Schneider M C: Preparation and properties of coenzyme A transferase. Received for publ. Oct. 28, 1955 in Enzymes of fatty acid metabolism IV.
Goldman D and Johnson M: Acetoacetate activation and cleavage enzyme system. Fed proc 12:209, 1953.
Stern J R, Coon M J and del Campillo A: Breakdown and synthesis of beta-keto fatty acids. In Enzymes of fatty acid metabolism III, pp 1-14, Rec for publ. Oct. 28, 1955.

One problem associated with total parenteral infusion techniques relates to infection of the catheter site, at times resulting in sepsis. The frequency of these secondary effects is described, for instance, by Snyderman et al (Total parenteral nutrition related infections. Prospective epidemiologic study using semiquantitative methods. Am J med. 73, 1982). Repeated blood cultivations showed that 12% of the patients examined had positive symptoms. Of this 12%, 5% had sepsis. The microbial contribution comprised, in diminishing frequency, Candida species, Staph epidermidis, Staph aureus and Serratia marcescens. It was possible to establish a relationship between the administration of fat emulsions through central or portal vein catheters and infection.

Another relationship between sepsis and central or portal vein catheters has been shown to reside in a prevailing infection at some other location in the body, for instance a urinary tract infection (Kovacevick et al: Association of parenteral nutrition catheter sepsis with urinary tract infections. JPEN 10(6):639-641, 1986. Weems J J, Chamberland M E, Ward J, Willy M, Padhye A A and Solomon S L: Candida parapsilosis fungemia associated with parenteral nutrition and contaminated blood pressure transducers. J Clin Microbiol 25(6):1029-1032, 1987). The same frequency of sepsis can also be established here.

Different endeavors are made in hospitals to avoid these complications. These endeavors have involved the administration of so-called all-in-one mixtures, in which all nutrients are blended and rapidly chilled, to avoid bacteria overgrowth before infusion can be administered (Jeppson et al: Bacterial growth properties in refrigerated all-in-one TPN mixtures. Clin Nutr 6:25-29, 1987), and also frequent replacement of the infusion set. In addition, it is generally forbidden to mix, for instance, preservatives with an infusion mixture containing fat emulsion. Furthermore, when working with fat emulsions, it is highly important to work aseptically on a sterile bench. Solutions containing fat emulsion have a highly restricted durability, among other things because of the microbiological contamination risk.

One method of counteracting the drawbacks of bacterial contamination and possible development of sepsis conditions with parenteral infusion in a vein, is to introduce an antimicrobial agent in conjunction with the infusion. However, it is generally forbidden to admix conventional preservatives with many of the fat emulsions involved.

It has now been found, however, that certain ketocarboxylic acids and their salts and esters exhibit strong antimicrobial properties against a pluraliaty of bacteria and fungi. Among these are included those bacteria and fungi which normally cause the aforesaid drawbacks, such as catheter sepsis, and a very good bactericidal effect has been obtained against, inter alia, Staphylococcus aureus, Escherichia coli and Candida albicans at proportions of the compound concerned as low as 0.2%.

Since the present compounds can also be considered to be potential energy substrates, they can be introduced in conjunction with parenteral nutrient administration, for instance in the form of an emulsion or as an additive to a nutrient solution. These compounds will then provide a nutrient boost, while at the same time exerting their microbiocidal effect.

The inventive compounds have also exhibited an antiviral effect. They can therefore also be used as agents against different virus.

Furthermore, the inventive compounds have also been found to have an effect on the central nervous system. This effect is primarily manifested as an anesthetic effect. The inventive compounds can therefore be used to influence the central nervous system, and then particu larly as an anesthetic.

The present invention thus relates to compounds suitable for use as nutrient substrates, as antimicrobial agents and antiviral agents, and/or as agents for influencing the central nervous system. The inventive compounds are characterised by the general formula

A - B

where A signifies a carboxylic acid group having a carbon chain which contains 3-10 carbon atoms, preferably 3-6 carbon atoms, and which is preferably branched and which contains one or more oxo groups bound to one or more carbon atoms in the chain, and B signifies a hydrogen atom, a pharmaceutically acceptable metal atom, an alcohol having 1-5 carbon atoms and 1-3 hydroxy groups bound as an ester, or a glycerol group bound as an ester.

The carboxylic acid group preferably contains one single oxo group which is bound to the carbon chain in the alpha or beta position of the carboxyl group.

A pharmaceutically acceptable metal atom comprises primarily an alkali metal atom or alkaline earth

metal atom, such as sodium, potassium, calcium or magnesium. Other non-toxic metals can also be used, however, as salt builders, and then particularly iron.

The alcohol bound as an ester preferably comprises a monoalcohol or glycerol. In the latter case, the acid may be bound to a primary or a secondary hydroxy group, and carboxylic acid groups may be bound to several hydroxy groups in the glycerol.

The following compounds are examples or particularly preferred inventive compounds:
4-methyl-3-oxo-pentanoic acid (beta-keto valinic acid)
5-methyl-3-oxo-hexanoic acid (beta-keto leucinic acid)
4-methyl-3-oxo-hexanoic acid (beta-keto isoleucinic acid)
isovaleric acid
pyruvic acid
alpha-keto valinic acid
butyryl acetic acid
and their alkali metal salts and/or ethyl esters.

The majority of the compounds according to the invention are previously known from the literature. However, it has not been known earlier that many of these compounds can be used as an energy substrate, or that they should have antimicrobial and antiviral activities, or activities on the central nervous system.

The preparation of the compounds according to the invention is also known from the literature, for instance from Jackman, M., Klenk, M., Fishburn, B., Tullar, B.F. and Archer, S.Ö "The preparation of some substituted thiohydantoins and thioimidazoles", J. Am. Soc. 70: 2884-2886 (1948) and others.

The inventive compounds can be formulated to different preparations for peroral, enteral or parenteral administration. Such compositions are also included in the present invention. These compounds may be included, in particular, in preparations for parenteral nutrient supply, primarily for intravenous administration.

The proportions in which the inventive compound or compounds is/are present in the preparations can vary within wide limits, and may be from 0.01 to 100 weight/volume percent. The specific proportion decided upon is determined by factors such as the composition and properties of the composition used, the effect desired, the method of administration and the patient's condition, etc. It lies within the competence of one skilled in this art to establish a suitable dosage against the background of the factors recited above.

Figure 1 of the accompanying drawings illustrates the results obtained when comparing nutrition experiments with the use of preparations containing compounds according to the invention.

Figure 2 illustrates the results obtained when testing the antiviral effects of the compounds.

Examples of how suitable preparations can be formulated are recited below.

One method of giving energy in the form of ethyl esters of branched beta-keto acids is to prepare an emulsion which includes beta-keto esters, phospholipids, glycerol, a basic amino acid and water. The emulsion may also include a greater or lesser quantity of vegetable oil. The reason for adding the basic amino acid (for instance lysine) is because if, for some reason or other, hydrolysis of the ester takes place, this will probably be followed by the release of carbon dioxide as a result of decarboxylation of liberated acid. This process is self-generating, i.e. if more acid is released, the hydrolysis will also increase as a result of diminishing pH, and so on. This process can thus be minimized by buffering the solution. Such a solution may comprise, for instance, 0.1-50% w/w of a pharmaceutically acceptable oil or fat, 0.02-20% of esters of branched beta-keto acids, and 0.01-6% w/w of phospholipids prepared from egg-yolk or soya. The emulsion may include, as further emulsion stabilizers to a total of 0.1-5% fatty acids having a chain length of 8-22 carbon atoms, their alkali metal salts or alkaline earth metal salts (for example $Ca^{++}$, $Mg^{++}$, $Na^{+}$, $K^{+}$. Isotonic substances, such as glycerol, glucose, fructose and buffering substances, such as different amino acids, primarily basic amino acids, such as lysine for example, can be included in the emulsion in amounts between 0.1-6% w/w. The substances recited, however, do not exclude the use of other isotonic substances or buffering substances.

An alternative method of administering beta-keto-ethyl esters is to dissolve phospholipid or some other parenterally acceptable emulsifier in the beta-keto-ethylester while heating the system, and then introducing a stablilizer, for instance ethyl alcohol. This solution can be sterilized and, with the aid of sterile-technique, can be transferred aseptically, for instance by injecting the solution through a sterile hypodermic syringe and needle into a fat emulsion, a glucose solution or amino-acid solution, either each per se or combined in a so-called all-in-one mixture. Such a solution can be obtained by dissolving in beta-keto-ethyl esters phospholipids prepared from egg-yolk or soya with heating. The amount can be varied from 0.1 to 10% w/w. This solution can then be stabilized, so as to prevent the phospholipide precipitating at room temperature, by using a solvent suitable for psyiological purposes, for instance ethyl alcohol. The amount can be varied from 0.1 to 50% w/w. This solution can then either be pasteurized at +80 °C over 3 hours, or autoclaved at sterilizing temperatures, suitably +117 °C for 20 minutes. A third method is one of using the

ester as such, which then forms an aggregate, or dissolving the ester in an aqueous solution, which may contain an emulsifier.

The previously mentioned branched beta-keto-esters have a high energy density. For example, 4-methyl-3-oxo-pentanoic acid ethyl ester has an energy density of 27.94 kJ/g, whereas the energy density for 5-methyl-3-oxo-hexanoic acid ethyl ester is 29.24 kJ/g.

The invention will now be described in greater detail, with reference to the following non-limiting examples.

Example 1

6.75 g of glycerol, 0.75 g of lysine and 7.2 g of egg-yolk phospholipids were added to 240 ml of sterile, distilled water. The dispersion was heated to +80 °C, while stirring. A mixture of 54 g refined soybean oil and 6 g of 4-methyl-3-oxo-pentanoic acid ethyl ester was added to the dispersion, whereafter the whole was homogenized in an Ultra-Turrax$^R$ homogenizer. The resultant coarse emulsion had a pH = 8.60 and was homogenized in a Manton-Gaulin-valve homogenizer (model M-15) partly at 15.6 MPa for 3 minutes and partly at 54.9 MPa for 6 minutes and finally at 15.6 MPa for 3 minutes. Subsequent to homogenization, the temperature was maintained at 50-58 °C with the aid of a cooler. The resultant emulsion was found to be stable when subjected to steam sterilization in glass flasks or bottles (+117 °C, 118 kPa for 20 min.). The emulsion was also found to be stable when shaken for 100 hours under standardized conditions. The emulsion contained 2% 4-methyl-3-oxo-pentanoic acid ethyl ester and 18% triglycerides from soybean oil.

Example 2

6.75 g of glycerol, 0.75 g of lysine and 7.2 g of egg-yolk phospholipids were added to 240 ml of sterile, distilled water. The dispersion was heated to +80 °C, while stirring. A mixture of 58.5 g of refined soybean oil and 1.5 g of 4-methyl-3-oxo-pentanoic acid ethyl ester was added to the dispersion, whereafter the whole was homogenized in an Ultra-Turrax$^R$ homogenizer. The resultant coarse emulsion had a pH = 8.60 and was homogenized in a Manton-Gaulin valve homogenizer (model M-15) partly at 15.6 MPa for 3 minutes and partly at 54.9 MPa for 6 minutes and finally at 15.6 MPa for 3 minutes. Subsequent to homogenization, the temperature was maintained at 50-58 °C with the aid of a cooler. The resultant emulsion was found to be stable when subjected to steam sterilization in glass flasks or bottles (+117 °C, 118 kPa for 20 min.). The emulsion was also found to be stable when shaken for 100 hours under standardized conditions. The emulsion contained 0.5% 4-methyl-3-oxo-pentanoic acid ethyl ester and 19.5% triglycerides from soybean oil.

Example 3

6.75 g of glycerol, 1.5 g of lysine and 7.2 g of egg-yolk phospholipids were added to 240 ml sterile distilled water. The dispersion was heated to +80 °C while stirring. A mixture of 54.0 g refined soybean oil and 6 g of 4-methyl-3-oxo-pentanoic acid ethyl ester was added to the dispersion, whereafter the whole was homo genized in an Ultra-Turrax$^R$ homogenizer. The resultant coarse emulsion had a pH = 9.50 and was homogenized in a Manton-Gaulin valve homogenizer (model M-15) partly at 15.6 MPa for 3 minutes and partly at 54.9 MPa for 6 minutes and finally at 15.6 MPa for 3 minutes. Subsequent to homogenization, the temperature was maintained at 50-58 °C with the aid of a cooler. The resultant emulsion was found to be stable when subjected to steam sterilization in glass flasks or bottles (+117 °C, 118 kPa for 20 min.). The emulsion was also found to be stable when shaken for 100 hours under standardized conditions. The emulsion contained 2% 4-methyl-3-oxo-pentanoic acid ethyl ester and 18% triglycerides from soybean oil.

Example 4

6.75 g of glycerol and 1.5 g of lysine and 7.2 g of egg-yolk phospholipids were added to 240 ml of sterile distilled water. The dispersion was heated to +80 °C, while stirring. A mixture of 58.5 g refined soybean oil and 1.5 g of 4-methyl-3-oxo-pentanoic acid ethyl ester was added to the dispersion, whereafter the whole was homogenized in an Ultra-Turrax$^R$ homogenizer. The resultant coarse emulsion had a pH = 9.0

and was homogenized in a Manton-Gaulin valve homogenizer (model M-15) partly at 15.6 MPa for 3 minutes and partly at 54.9 MPa for 6 minutes and finally at 15.6 MPa for 3 minutes. Subsequent to homogenization, the temperature was maintained at 50-58 °C with the aid of a cooler. The resultant emulsion was found to be stable when subjected to steam sterilization in glass flasks or bottles (+117 °C, 118 kPa for 20 min.). The emulsion was also found to be stable when shaken for 100 hours under standardized conditions. The emulsion contained 0.5% 4-methyl-3-oxo- pentanoic acid ethyl ester and 19.5% triglycerides from soybean oil.

Example 5

A so-called mother concentrate was prepared by dissolving 6 g of egg-yolk phospholipids in 40 g of 4-methyl-3-oxo-pentanoic acid ethyl ester at +60 °C, while stirring. 34 g of 99% ethyl alcohol were then added to the solution, whereafter the solution was cooled to room temperature and dispensed into 20 ml glass flasks. Nitrogen gas was then bubbled through the liquid and the flasks then stoppered and capsuled. The bottles were then autoclaved at +120 °C and at 118 kPa for 20 minutes. A 10% fat emulsion for parenteral nutrient administration (Intralipid^R retailed by KabiVitrum AB, Stockholm) was added to the preparation under strict asceptic conditions, to a 4-methyl-3-oxo-pentanoic acid ethyl ester concentration of 0.2, 1 and 3% respectively. The emulsion was shaken for 5 minutes in a shaking machine and then placed in a cold environment (+5 °C). The emulsion was thereafter observed over a 5 day period, wherewith a daily inspection was carried out to assess the formation of oil droplets, creaming or oil separation. The assessment was made in comparison with a known emulsion for parenteral use (Intralipid^R 10%). It was found that the additions of 0.3% and 1% had no affect on the quality over the 5 days during which the test was carried out in comparison with Intralipid^R 10% lacking said additions. During the first three days, the emulsion containing the 3% addition was fully acceptable, but deteriorated slowly during day 4 and 5. Subsequent to 5 days storage, an analysis was made of the pH and mean particle size in a Coulter nanosizer. The following values were obtained:

| | 3% admix | 1% admix | 0.2% admix | 0% admix |
|---|---|---|---|---|
| pH | 6.8 | 7.0 | 7.2 | 7.9 |
| Mean particle size | 264 | 245 | 258 | 242 |
| | 264 | 253 | 264 | 245 |
| | 264 | 250 | 267 | 248 |
| | 258 | 235 | 250 | |
| | 248 | 245 | 264 | |
| | 250 | 250 | | |
| | | 242 | | |
| $\overline{X}$ | $\overline{258}$ | $\overline{246}$ | $\overline{261}$ | $\overline{245}$ |
| S.D. | 7.4 | 6.0 | 6.8 | 3 |

The above results indicate that 4-methyl-3-oxo-pentanoic acid ethyl ester is either dissolved into an existing fat emulsion or forms its own emulsion of "normal"-micro type or liposome type. Emulsification, or incorporation, takes place very quickly, with the minimum of energy consumption. The experiments illustrate a novel method of preparing and administering a hydrophobic substance under asceptic conditions, without needing to prepare a totally new emulsion.

Example 6

Unspecified toxicity was tested on four mice by first weighing the animals, and then injecting the animals intravenously with 0.5 ml of an emulsion of 4-methyl-3-oxo-pentanoic acid having a concentration of 50 mg/ml for 10 minutes. Each mouse received a dosage of 25 mg. The emulsion was prepared aseptically, by emulsifying sterile-filtered ester with a sterilized dispersion of egg-yolk phospholipids. The animals were not seen to react to the injection to any marked extent, except for a certain lowering of the respiration frequency, which was compensated for by lowering the dosage administration rate (resulting in longer

infusion time). Neither was any abnormal change in the animals' behaviour observed during the following 7 days. The increase in weight of the animals was normal during the observation period (weight increase: X = 4.5 g, S.D. 0.5).

Example 7

The unspecific toxicity was tested on 5 mice by first weighing the animals, and then injecting intravenously 0.5 ml of an emulsion of 4-methy-3-oxo-pentanoic acid ethyl ester having a concentration of 5 mg/ml for 15 seconds. The emulsion was aseptically prepared, by emulsifying sterile-filtered ester with a sterilized dispersion of egg-yolk phospholipids. The animals showed no marked reaction during the injection. Each mouse received a total dosage of 2.5 mg. Neither did the animals show any abnormal behaviour during the following 7 days.

Example 8

Unspecified toxicity was tested on four mice by first weighing the animals, and then injecting intravenously 0.2 ml of an emulsion of 4-methyl-3-oxo-pentanoic acid ethyl ester having a concentration of 33 mg/ml for 30 seconds. Each mouse received a dosage of 6.7 mg. The emulsion was prepared aseptically, by emulsifying sterile-filtered ester with a sterilized dispersion of egg-yolk phospholipids. The animals lost consciousness after being administered with roughly half the aforesaid dose. The animals exhibited symptoms of deep anesthesia, including, inter alia, respiratory depression, pupil contraction and weak cardiac rhythm. The animals suddenly recovered consciousness about 45 seconds after completing the injection, and were unaffected. No abnormal behaviour was observed in the animals during the following 7 days. The increase in weight of the animals was normal during the observation period (X = 4.6, S.D. = 1.1).

Example 9

Unspecified toxicity was tested on five mice, by first weighing the animals and then injecting intravenously 0.5 ml of an emulsion of 5-methyl-3-oxo-hexanoic acid ethyl ester having a concentrtion of 5 mg/ml over a period of 15 seconds. Each mouse received a dosage of 2.5 mg. The emulsion was prepared aseptically, by emulsifying sterile filtered ester with a sterilized dispersion of egg-yolk phospholipids. The animals became unconscious after receiving approximately half the aforesaid dosage. The animals exhibited the symptom of being under an anesthetic, and had, inter alia, respiratory depression, pupil contraction and weak cardiac rhythm. The animals regained consciousness about 10 seconds after completing the injection, and breathed heavily, and after 35 seconds, appeared quite normal. No abnormal behaviour could be observed in the animals during the next following 7 days. The increase in weight of the animals was normal during the observation period (X = 5.0, S.D. = 1.1).

Example 10

Two rabbits weighing between 3.0-3.5 kg were infused intravenously (10 ml/kg body weight) with an emulsion containing 50 mg/ml of 4-methyl-3-oxo-pentanoic acid ethyl ester over a period of 5 minutes. Each rabbit received a dosage of 1.5-1.8 g over a 5 minute period. The emulsion was prepared aseptically, by emusifying sterile-filtered ester with a sterilized dispersion of egg-yolk phospholipids. The animals lost consciousness during the infusion process, after having received approximately 1/3 of the aforesaid dosage. The animals exhibited symptoms of deep anesthesia including, inter alia, respiratory depression, pupil contraction and weak cardiac rhythm. The animals regained consciousness about 2 minutes after completion of the infusion, and then exhibited normal behaviour patterns.

Example 11

Three rabbits weighing 3.0-3.3 kg were infused intravenously (10 ml/kg body weight) with an emulsion containing 30 mg/ml of 4-methyl-3-oxo-pentanoic acid ethyl ester over a period of from 4 to 7 minutes.

Each rabbit received a dosage of 0.9-1.05 g. The emulsion was prepared aseptically, by emulsifying sterile-filtered ester with a sterilized dispersion of egg-yolk phospholipids. During the infusion process, two of the animals lost consciousness subsequent to receiving approximately 4/5 of the aforesaid dosage. As with the earlier examples, the animals exhibited a symptom indicative of anesthesia including, inter alia, respiratory depression, pupil contraction and weak cardiac rhythm. The third rabbit remained normal during the whole of the infusion period. The two unconscious rabbits awakened about 2 minutes after completion of the infusion, and thereafter acted quite normally.

Example 12

Two 20%-emulsions containing 4-methyl-3-oxo-pentanoic acid ethyl ester and 5-methyl-3-oxo-hexanoic acid ethyl ester respectively were prepared. In addition to ester, the emulsions also comprised egg-yolk phospholipid 1,2% and sterile water. The emulsions were prepared aseptically, by emulsifying sterile-filtered ester with a sterilized dispersion of egg-yolk phospholipids. Aliquots of these emulsions were diluted with a phosphate buffer (0.01 M, pH = 7.0) in the proportions of 1 and 10 respectively, so as to obtain 2-percent emulsions. Various microorganisms according to Table 1 below were added to these emulsions. The samples were then incubated at +37° C for 20 hours. Counting of the number of microorganisms was started after 2 and 20 hours, in the manner described in microbiological literature. Checks were run on a pure phosphate buffer with additives of the same microorganisms as earlier, in parallel with the aforesaid experiments, while checks were run in Tryptone Soya broth (TSB) with additions of the same microorganisms, in order to ascertain whether or not the microorganisms could survive, i.e. could grow under the ideal conditions. The microorganism count at different points in time is presented in Table 1. The result shows that both 4-methyl-3-oxo-pentanoic acid ethyl ester and 5-methyl-3-oxo-hexanoic acid ethyl ester at a concentration of 2% have good bacteria inhibiting properties over a broad spectrum of species, good yeast inhibition and, in the case of certain species, were effective in killing $10^3$ cells within 20 hours.

TABLE 1

| | | 4-methyl-3-oxo-pentanic acid-ethyl ester | | 5-methyl-3-oxo-hexanoic acid-ethyl ester | | phosphate buffer | TSB |
|---|---|---|---|---|---|---|---|
| Strain | Additive/ml | 2 hrs | 20 hrs | 2 hrs | 20 hrs | 20 hrs | 20 hrs |
| Staf. aureus ATCC 6535 | 5012 | 0 | 0 | 0 | 0 | 631 | $6.3x10^7$ |
| Staf. epiderm. | 6310 | 0 | 0 | 794 | 25 | 25 | $10^9$ |
| E. coli ATCC 8739 | 6310 | 0 | 0 | 0 | 0 | 6310 | $6.3x10^8$ |
| Klebs. pneum. ATCC 25955 | 1995 | 0 | 0 | 0 | 0 | 1995 | $10^9$ |
| Serr. maresc. ATCC 14756 | 6310 | 0 | 0 | 0 | 0 | $2.5x10^4$ | $2x10^9$ |
| Proteus vulg. ATCC 13315 | 1000 | 0 | 0 | 0 | 0 | 2000 | $6.3x10^8$ |
| Ps. aeruginosa ATCC 19429 | 7943 | 0 | 0 | 0 | 0 | $7.9x10^4$ | $1.3x10^9$ |
| Enter. cloacae | 1585 | 0 | 0 | 0 | 0 | 1585 | $7.9x10^8$ |
| Cand. albicans ATCC 10231 (yeast) | 6310 | 200 | 0 | 1995 | 0 | $2x10^4$ | $4.0x10^6$ |
| Cand. parapsil. (yeast) | 5012 | 1000 | 0 | 3162 | 0 | 7943 | $1.3x10^6$ |
| Asperg. niger ATCC 16404 (mould) | 7943 | 3981 | 1000 | 5012 | 5012 | 6310 | 3162 |
| Penicillium sp. (mould) | 3162 | 5012 | 0 | 3981 | 316 | 3162 | 3981 |
| Ulocladium sp. (mould) | 2512 | 3981 | 0 | 5012 | 0 | 3162 | 1000 |

Example 13

Two 20% emulsions containing respectively 4-methyl-3-oxo-pentanoic acid ethyl ester and 5-methyl-3-oxo-hexanoic acid ethyl ester were prepared. Both of the emulsions included egg-yolk phospholipide 1,2% and sterile water, in addition to ester. The emulsions were prepared aseptically, by emulsifying sterile-filtered ester with a sterilized dispersion of egg-yolk phospholipids.

Aliquots of these emulsions were diluted with phosphate buffer (0.01 M, pH = 7.0), in the proportions of 1 and 100 in respective emulsions. Staphylococcus aureus ATCC 6535 was then added to the emulsions. The samples were then incubated at +37 °C for 20 hours. The microorganism count was carried out after 1-5 minutes, 2 and 20 hours, in the manner described in microbiological literature. Parallel with the above experiments, a check was made on the phospholipid solution 1,2% and TSB (Tryptone soya broth), by adding Staphylococcus aureus ATCC 6535. The latter substrate was tested to ascertain whether the controlled microorganisms were viable. After 20 hours, the number of organisms/ml in TSB were found to be $10^8$, and in the phospholipide solution $7x10^2$. The starting concentration was about $5x10^3$. After 1-5 minutes, the number of microorganisms present in the samples containing 4-methyl-3-oxo-pentanoic acid ethyl ester with concentrations 2% and 0.2% were found to be 100 and $10^4$ respectively. After 2 hours, the number of microorganisms was 0 and 10 respectively, and after 20 hours 0 and 0 respectively. In the samples containing 5-methyl-3-oxo-hexanoic acid ethyl ester with concentrations of 2% and 0.2%, the number of microorganisms per ml present after 1-5 minutes was $10^4$, after 2 hours 0 and 100 respectively, and after 20 hours 0 and 0 respec tively. The experiment showed that a concentration of 0.2% of 4-methyl-3-oxo-pentanoic acid ethyl ester and 5-methyl-3-oxo-hexanoic acid ethyl ester were also effective to kill Staphylococcus aureus.

Example 14

Two 20% emulsions containing 4-methyl-3-oxo-hexanoic acid ethyl ester and 5-methyl-3-oxo-hexanoic acid ethyl ester were prepared. The emulsions contained egg-yolk phospholipid 1,2% and sterile water, in addition to ester. The emulsions were prepared aseptically, by emulsifying sterile-filtered ester with a steriled dispersion of egg-yolk phospholipids.

Aliquots of these emulsions were diluted with phosphate buffer (0.01 M, pH = 7.0) in the proportions of 1 and 10 and 1 and 100 in respective emulsions. Escherichia coli ATCC 8739 was added to the solutions. The solutions were then incubated at +37 °C for 20 hours. The microorganism count was carried out after 1-5 minutes, 2 and 20 hours, in the manner described in microbiological literature. Parallel with the above tests, a check was carried out on phospholipide solution 1,2% and TSB (Tryptone soya broth) with additions of Escherichia coli ATCC 8739. The latter substrate was tested to ascertain whether or not the control microorganisms were viable. After 20 hours, the number of organisms/ml in TSB was found to be $10^9$, and in the phospholipid solution also $10^9$. The starting concentration was about $10^4$. In the samples containing 4-methyl-3-oxo-pentanoic acid ethyl ester having the concentrations of 2% and 0.2%, the number of microorganisms present per ml after 1-5 minutes was 0 and $10^4$ respectively. After 2 hours, the microorganism count was 0 and 10 respectively, and after 20 hours 0 and 10 respectively. In the samples containing 5-methyl-3-oxo-hexanoic acid ethyl ester with the concentrations of 2% and 0.2%, the microorganism count per ml was 10 and 10 respectively after 1-5 minutes, 0 and 0 respectively after 2 hours, and 0 and 0 respectively after 20 hours. The test also showed that a concentration of 0.2% of 4-methyl-3-oxo-pentanoic acid ethyl ester and 5-methyl-3-oxo-hexanoic acid ethyl ester were effective to kill Escherichia coli.

Example 15

Two 20% emulsions containing 4-methyl-3-oxo-pentanoic acid ethyl ester and 5-methyl-3-oxo-hexanoic acid ethyl ester respectively were prepared. The emulsions contained egg-yolk phospholipid 1,2% and sterile water, in addition to ester. The emulsions were prepared aseptically, by emulsifying sterile-filtered ester with a sterilized dispersion of egg-yolk phospholipids.

Aliquots of these emulsions were diluted with phosphate buffer (0.01 M, pH = 7.0) in the proportions of 1

and 10 and 1 and 100 of respective emulsions. Candida albicans ATCC 10231 was added to the emulsions. The samples were then incubated at +37° C for 20 hours. The microorganism count was carried out after 15 minutes, 2 and 20 hours, in the manner described in microbiological literature. Parallel with the above tests, a check was made on phospholipide solution 1,2% and TSB (Tryptone soya broth) with additions Candida albicans ATCC 10231. The latter substrate was tested to ascertain whether or not the microorganisms were viable. After 20 hours, the microorganism count/ml in TSB was $10^7$, and in the phospholipid solution $10^4$. The starting concentration was about $10^4$. After 1-5 minutes, the microorganism count per litre of the tests containing 4-methyl-3-oxo-pentanoic acid ethyl ester with concentrations of 2% and 0.2% was $10^4$ and $10^4$ respectively. After 2 hours, the microorganism count was $10^2$ and $10^4$ respectively, and after 20 hours 0 and $10^4$. In the samples containing 5-methyl-3-oxo-hexanoic acid ethyl ester with the concentrations of 2% and 0.2%, the microorganism count per ml of sample was found to be $10^4$ after 1-5 minutes, and $10^3$ and $10^4$ respectively after 2 hours. After 20 hours, the count was 0 and $10^2$ respectively. The test shows that a concentration of 0.2% of 4-methyl-3-oxo-pentanoic acid ethyl ester is also effective in prohibiting growth of Candida albicans, whereas a concentration of 0.2% of 5-methyl-3-oxo-hexanoic acid ethyl ester will reduce the number of fungi within 20 hours.

## Example 16

Two 20% emulsions containing 4-methyl-3-oxo-pentanoic acid ethyl ester and 5-methyl-3-oxo-hexanoic acid ethyl ester were prepared. The emulsions contained egg-yolk phospholipid 1,2% and sterile water in addition to ester. The emulsions were prepared aseptically, by emulsifying sterile-filtered ester with a sterilized dispersion of egg-yolk phospholipids.

Aliquots of these emulsions were diluted at a ratio of 1 to 10 with phosphate buffer (0.01 M, pH = 7.0), seruma albumin solution 20% and Tryptone soya broth (TSB) respectively. Escherichia coli ATCC 8739 were added to the emulsions. The emulsions were then incubated for 20 hours at +37° C. The microorganism count was carried out after 1-5 minutes, 2 hours and 20 hours, in the manner described in microbiological literature. Parallel with the above tests, there was made a control of phosphate buffer and TSB with additions of Escherichia coli ATCC 8739. The latter substrate was tested to ascertain whether or not the microorganisms were viable. After 20 hours, the number of microorganisms present in TSB was found to be $10^4$; in the phosphate buffer the microorganism count was $10^4$. The starting concentration was about $10^4$. In the samples containing 4-methyl-3-oxo-pentanoic acid ethyl ester, the combination with phosphate buffer was 0 after 5 minutes, 0 after 2 hours and 0 after 20 hours. In the combination with 20% albumin solution, the microorganism count was 0 after 5 minutes, no count was taken after 2 hours, and 0 after 20 hours. After 4 hours, the count in the combination with TSB was 0, no count was taken after 2 hours, and 0 after 20 hours.

In the samples containing 5-methyl-3-oxo-hexanoic acid ethyl ester, the count in the combination with phosphate buffer was 10 after 5 minutes, 0 after 2 hours, and 0 after 20 hours. The combination with 20% albumin solution was $10^2$ after 5 minutes, no count was taken after 2 hours, and 0 after 20 hours. In the combination with TSB, the count was 10 after 5 minutes, no count was taken after 2 hours, and 0 after 20 hours. The test showed that, even under the most favourable growth conditions, all bacteria were killed within 5 minutes in the presence of 4-methyl-3-oxo-pentanoic acid ethyl ester, and that a pronounced reduction was obtained in the presence of 5-methyl-3-oxo-hexanoic acid ethyl ester. All microorganisms were eliminated within 20 hours by both esters at the same low concentration.

## Example 17

27.0 g of 5-methyl-3-oxo-hexanoic acid ethyl ester were mixed with 316 ml of 0.5 M sodium hydroxide solution and 150 ml of absolute alcohol, while stirring. The reaction was interrupted after 23 hours, by adding 1.0 M hydrochloric acid to the reaction mixture to a pH = 9.25. The solution was then evaporated to about 100 ml and freeze-dried in accordance with conventional techniques over 24 hours. A white powder was obtained. The powder weighed 21.5 g, which corresponds to a yield of 82.3%. The identity and purity was confirmed by NMR, IR and HPLC.

## Example 18

25.7 g of 4-methyl-3-oxo-pentanoic acid ethyl ester were mixed with 316 ml of 0.5 M sodium hydroxide solution and 150 ml of absolute alcohol, while stirring. After 22 hours, the solution had a pH of 8.58. The solution was evaporated to 100 ml and freeze-dried in accordance with conventional techniques, for 24 hours. A white powder was obtained. The powder weighed 21.4 g, which corresponds to a yield of 86.6%. The identity and purety were confirmed by NMR, IR and HPLC.

Example 19

Two solutions containing 10% of the sodium salts of 4-methyl-3-oxo-pentanoic acid ethyl ester and 5-methyl-3-oxo-hexanoic acid ethyl ester respectively of pH = 7.30 were adjusted with 1 M hydrochloric acid, diluted 1/5 with phosphate buffer (0.01 M, pH = 7.00), such as to obtain 2-percent solutions. Staphylococcus epidermidis 61540-51 and Escherichia coli ATTC 8739 were each added to a respective solution in a concentration of about 3000-10 000/ml. These samples were then incubated at +37° C for 20 hours. A microorganism count was carried out after 2 and 20 hours, in a manner described in microbiological literature. Parallel with the aforesaid tests, a control was run in pure phosphate buffer and in Tryptone soya broth (TSB) with additions of Staphylococcus epidermidis 61540-51 and Escherichia coli ATTC 8739 respectively, to ascertain the viability of the microorganisms to survive, i.e. the ability of the microorganisms to grow under the ideal conditions provided. After 2 hours, the number of organisms/ml of type Staphylococcus epidermidis in the phosphate solution was 4300 and after 20 hours 1. Growth could be established in TSB after 20 hours. No organisms were found in the solution containing 2%-sodium salt of 4-methyl-3-oxo-pentanoic acid ethyl ester after both 2 and 20 hours. This was also the case with the sodium salt of 2 percent 5-methyl-3-oxo-hexanoic acid ethyl ester. After 2 hours, the microorganism count of Escherichia coli in the phosphate solution was 3400, and after 20 hours 7400. Growth was established in TSB after 20 hours. In the solution containing 2% sodium salt of 4-methyl-3-oxo-pentanoic acid ethyl ester, the organism count was 2000 after 2 hours and 0 after 20 hours. With respect to the sodium salt of 5-methyl-3-oxo-hexanoic acid ethyl ester, the organism count was 44 after 2 hours and 0 after 20 hours. The results show that the tested sodium salts at pH = 7 rapidly killed the experimental bacteria.

Example 20

The following emulsions were prepared. 20% ethyl-isovalerate, pH = 8.7. Ethyl pyruvate 17%, pH = 5.7, ethyl propionylacetate 4%, pH = 6.70; ethyl-3-methyl-2-oxo-butyrate 8%, pH = 5.00; ethyl caproate 20%, pH = 8.92; ethyl butyrylacetate 20%, pH = 7.50; ethyl acetoacetate 17%, pH = 6.50; 20% triglyceride which included 4% 5-methyl-3-oxo-hexanoic acid bound in 1 and/or 3-position, pH = 7.70. All emulsions were diluted down to 2%, with a phosphate buffer (PBS) 0.01 M, pH = 7.00. Staphylococcus epidermidis 61540-51 and Candida albicans ATCC 10231 were added to the diluted emulsions and incubated at +37° C for 20 hours. Samples were taken after some minutes, after 2 hours and then after 20 hours, for the purpose of counting the number of microorganisms present in the manner described in microbiological literature. Parallel with the aforesaid tests, controls were carried out in pure PBS and Tryptone soya broth (TSB) with additions of the aforesaid microorganisms. The result of the tests is shown in Table 2.

TABLE 2

| | Staphylococcus epidermis | | | Candida albicans | | | E. coli | | |
|---|---|---|---|---|---|---|---|---|---|
| | 0 hr | 2 hrs | 20 hrs | 0 hr | 2 hrs | 20 hrs | 0 hr | 2 hrs | 20 hrs |
| PBS | 8500 | 4300 | 1 | 5300 | 4400 | 5100 | 2800 | 3400 | 7400 |
| TSB | | | turb. | | | turb. | | | turb. |
| 4-methyl-3-oxo hexanoic acid-ethyl ester | 490 | 0 | 0 | 3700 | 3000 | 0 | 480 | 0 | 0 |
| Ethyl-isovalerate | 7300 | 0 | 0 | 370* | 1 | 0 | 23* | 0 | 0 |
| Ethyl-pyruvate | 4100 | 0 | 0 | 3800 | 0 | 0 | 0* | 0 | 0 |
| Ethyl-propionylacetate | 11000 | 200 | 12 | 4500 | 3800 | 0 | 7900 | 22 | 0 |
| Ethyl-3-methyl-2-oxo-butyrate | 1* | 0 | 0 | 4100 | 7 | 0 | 0* | 0 | 0 |
| Ethyl-caproate | 9400 | 27 | 1 | 1100 | 32 | 0 | 65* | 0 | 0 |
| Ethyl-butyrylacetate | 4000 | 0 | 0 | 5800 | 220 | 0 | 1* | 0 | 0 |
| Ethyl-acetoacetate | 7700 | 4 | 0 | 4500 | 4200 | 0 | 3900 | 2400 | 40 |
| Triglyceride with 5-methyl-3-oxo-hexanoic acid | 9500 | 2400 | 3700 | 3900 | 4700 | 14500 | 3000 | 3800 | >3000 |

* The killing ability was probably so high that there was no time to take an 0-sample with living organisms.

## Example 21

Unspecified toxicity was tested on 5 mice, by weighing the animals and then injecting intravenously 0.5 ml of an 8 percent solution (pH = 7.0) of the sodium salt of 4-methyl-3-oxo-pentanoic acid ethyl ester over a period of 30 seconds. Each mouse received a dosage of 40 mg. Dyspnea and cyanosis were observed during the injection period. One minute after the completion of the injection, each animal returned to normal. All animals exhibited normal weight development over the following 7 days.

## Example 22

Unspecified toxicity was tested on 5 mice, by weighing the animals and injecting intravenously 0.4 ml of a 7.5%-solution (pH = 7.00) of the sodium salt of 5-methyl-3-oxo-hexanoic acid ethyl ester over a 30 second period. Each mouse received a dose of 37 mg. Dyspnea and cyanosis were oberved during the injection period. Each animal returned to normal 1 minute after completing the injection. All animals exhibited thereafter normal weight development over the following 7 days.

## Example 23

Unspecified toxicity was tested on five mice, by weighing the animals and injecting intravenously over a 30 second period 0.2 ml of a 20-percent emulsion (pH = 7.5) containing bound 5-methyl-3-oxo-hexanoic acid corresponding to about 22% of the fat component. The acid was bound by transesterification in the 1- and 3-position to the triglyceride derived from pure soybean oil. The remainder of the fat component comprised 5-methyl-3-oxo- hexanoic acid ethyl ester (about 9%), ethyl esters of different fatty acids derived from the soybean oil and unchanged triglycerides from the soybean oil. The fat components were emulsified together with phospholipides, glycerol and lysine in water phase in accordance with conventional methodology. The glycerides containing bound 5-methyl-3-oxo-hexanoic acid were administered in dosages of 8-10 mg per mouse, while the dosage of free 5-methyl-3-oxo-hexanoic acid ethyl ester was about 4 mg per mouse. Dyspnea and cyanosis were observed during the injection period. These symptoms disappeared one minute after completion of the injection and the animals behaved normally thereafter. Weight development of all animals was normal during the following seven days.

Example 24

39.3 g of 5-methyl-3-oxo-hexanoic acid-ethyl ester was dissolved in 100.0 g of pure soybean oil, while in a nitrogen gas atmosphere at +60 °C, while stirring the system. 0.9 ml of distilled water and 14 g of Lipozyme[R] were added to the solution. (Lipozyme[R] is a commercially available lipid-cleaving enzyme produced by Novo Industrier, Copenhagen, Denmark, this enzyme being of microbiological origin and cleaving specifically at positions 1 and 3 of the triglycerides). The reaction continued over a period of 4 calender days, whereafter the enzyme was filtered off and the crude product washed with 1% sodium hydrogen carbonate solution. The product was found to contain about 9% free 5-methyl-3-oxo-hexanoic acid ethyl ester. The ester-bound acid (as triglyceride ester) was calculated to be approximately 22% of the total amount of triglycerides.

Example 25

39.2 g of 5-methyl-3-oxo-hexanoic acid ethyl ester were dissolved in 100.2 g of pure soybean oil, under a nitrogen-gas atmosphere at +50 °C, while stirring the system. 14 g of Lipozyme[R] and 0.9 ml of distilled water were added to the solution. The reaction continued for four calender days, whereafter the enzyme was filtered off and the crude product distilled at +97 °C and 4-10 mm Hg to constant weight. The distillation process was repeated three times subsequent to small additions of ethanol. The intention was to distill off any remaining non-reacted ethyl esters of 5-methyl-3-oxo-hexanoic acid. It was possible to remove 9.1% of the original amount of ester in this way. The remainder, i.e. 90.8% of the original amount of 5-methyl-3-oxo-hexanoic acid ethyl ester was thus incorporated in glycerides. The proportion of novel glyceride in the product could therefore be calculated to 49.4% (not corrected for free fatty acids, which corresponded to about 5%).

Example 26

39.8 g of 4-methyl-3-oxo-pentanoic acid ethyl ester were dissolved in 101.5 g of pure soybean oil in a nitrogen-gas atmosphere at +60 °C, while stirring the system. 14 g of Lipozyme[R] and 0.9 ml of distilled water were added to the solution. The reaction continued for four calender days, whereafter the enzyme was filtered off and the crude product distilled at +97 °C and 4-10 mm Hg to constant weight. The intention was to distill off any remaining non-reacted ethyl esters of 4-methyl-3-oxo-pentanoic acid. It was possible to remove 8.3% of the original amount of ester in this way. The remainder, i.e. 91.7% of the original quantity of 4-methyl-3-oxo- pentanoic acid ethyl ester was thus incorporated in glycerides. The proportion of novel glyceride in the product could therefore be calculated to 55.8% (not corrected for the quantity of free fatty acids, which corresponded to about 5%).

Example 27

Unspecified toxicity was tested on five mice, by weighing the animals and then injecting intravenously over a period of 1 minute 0.5 ml of an emulsion of 4-methyl-3-oxo-hexanoic acid ethyl ester having a concentration of 200 mg/ml. The emulsion was prepared aseptically, by emulsifying sterile-filtered ester with a sterilized dispersion of egg-yolk phospholipids. During the process of injection, dyspnea and coma were observed. These symptoms ceased within 2 minutes of completing the injection, and the majority of symptoms had disappeared after a further 2 minutes. Some of the animals, however, exhibited itching-like symptoms from the outermost tips of their tails, over a further few minutes. All of the animals appeared to be normal 7 minutes after completion of the injection, and continued to behave normally over a subsequent 7-day observation period. The weight increase over this period was also found to be normal ($X = 3.1 ^{+} 0.8$ g). Each animal received a total dosage of 100 mg.

Example 28

6.75 g of glycerol, 0.75 g of lysine and 7.2 g of egg-yolk phospholipids were added to 240 ml of sterile distilled water. The mixture was heated to +8 °C, while stirring the system. 48 g of refined soybean oil and

12 g of 4-methyl-3-oxo-pentanoic acid ethyl ester were added to the mixture. The mixture was homogenized in an Ultra-Turrax[R] homogenizer. The resultant coarse emulsion, having a pH = 8.00, was homgenized in a Manton-Gaulin valve homgenizer (model M-15) partly at 14.7 MPa over 3 minutes, partly at 54.9 MPa over 6 minutes, and finally at 15.6 MPa over 3 minutes. Subsequent to homogenization, the temperature was maintained at 50-58° C with the aid of a cooler. The resultant emulsion was stable when steam sterilized in glass flasks (+117° C, 118 kPa for 20 minutes). The emulsation contained 4% 4-methyl-3-oxo-pentanoic acid-ethyl ester and 16% triglycerides from soya oil.

Example 29

240 ml of sterile distilled water was admixed with 6.75 g of glycerol, 0.75 g of lysine and 7.2 g of egg-yolk phospholipids. The mixture was heated to +80° C, while stirring the system. 49.5 g of refined soybean oil and 10.5 g of 5-methyl-3-oxo-hexanoic acid ethyl ester were added to the mixture. The mixture was then homogenized in a Ultra-Turrax[R] homogenizer. The resultant coarse emulsion, pH = 8.6, was homogenized in a Manton-Gaulin valve homogenizer (model M-15), partly at 15.6 MPa for 3 minutes, partly at 54.9 MPa for 6 minutes, and finally at 15.6 MPa for 3 minutes. Subsequent to homogenization, the temperature was maintained at 50-58° C with the aid of a cooler. The resultant emulsion was found to be stable when steam sterilized in glass flasks (+117° C, 118 kPa for 20 minutes). The emulsicn contained 3.5% 5-methyl-3-oxo-hexanoic acid ethyl ester and 16.5% triglycerides from soybean oil.

Example 30

A so-called mother concentrate produced by dissolving 6 g egg-yolk phospholipids in 40 g of 4-methyl-3-oxo-pentanoic acid-ethyl ester at +60° C, while stirring the system. 34 g of 99% ethyl alcohol were then added to the solution, whereafter the solution was cooled to room temperature and dispensed into 20 ml glass flasks. Nitrogen gas was perculated through the contents of the flasks, and the flasks subsequently stoppered and fitted with capsules. The flasks were then autoclaved at 120° C and 118 kPa over a period of 20 minutes. This preparation was added to a 20% fat emulsion (Intralipid[R]) under strict aseptic conditions, such that the concentation of 4-methyl-3-oxo-pentanoic acid ethyl ester was 0.2 and 1 and 3% respectively. The emulsion was shaken for 5 minutes in a shaker and then placed in a cold environment (5° C). The emulsion was inspected over a period of 5 calender days, wherewith a visual inspection was carried out daily in order to ascertain the occurrence of any oil droplets that may form, creaming or oil separation. The assay was effected by comparison with a known emulsion for parenteral use (Intralipid[R] 20%). No change in quality was noticed over the 5 test-day period with respect to the 0.2% and 1% additions, compared with Intralipid[R] 20% lacking such addition. The emulsion containing the 3-percent mixture was found to be fully acceptable over the first 3 days, but slowly deteriorated during day 4 and day 5. The pH of the emulsion was measured after a 5 day storage period. The mean particle size was measured in a Coulter[R] nanosizer. The following values were obtained:

| | 3% admix | 1% admix | 0.2% admix | 0% admix |
|---|---|---|---|---|
| pH | 6.5 | 6.9 | 7.3 | 7.7 |
| Mean particle size | 322 | 323 | 325 | 287 |
| | 319 | 323 | 325 | 316 |
| | 320 | 327 | 316 | 330 |
| | 333 | 329 | 320 | 313 |
| | 319 | 319 | 327 | 320 |
| | | 336 | | |
| $\overline{X}$ | $\overline{323}$ | $\overline{326}$ | $\overline{323}$ | $\overline{313}$ |
| S.D. | 5.9 | 5.9 | 4.5 | 16.0 |

The above results indicate that 4-methyl-3-oxo-pentanoic acid ethyl ester either dissolves into an existing fat emulsion or forms its own emulsion of "normal" microtype or liposomee type. The emulsifying or incorporating process takes place over a very short time period, with minimum energy consumption. The

experiments provide a novel method of preparing and supplying a hydrophobic substance under aseptic conditions, without needing to prepare a completely new emulsion.

Example 31

A so-called mother concentrate produced by dissolving 6 g egg-yolk phospholipids in 40 g of 4-methyl-3-oxo-pentanoic acid-ethyl ester at +60° C, while stirring the system. 34 g of 99% ethyl alcohol were then added to the solution, whereafter the solution was cooled to room temperature and dispensed into 20 ml glass flasks. Nitrogen gas was perculated through the contents of the flasks, and the flasks subsequently stoppered and fitted with capsules. The flasks were then autoclaved at 120° C and 118 kPa over a period of 20 minutes. This prepara tion was added to a 10% fat emulsion (Intralipid$^R$) under strict aseptic conditions, such that the concentation of 4-methyl-3-oxo-pentanoic acid ethyl ester was 0.2 and 1 and 3% respectively. The emulsion was shaken for 5 minutes in a shaker and then placed in a cold environment (5° C). The emulsion was inspected over a period of 5 calender days, wherewith a visual inspection was carried out daily in order to ascertain the occurrence of any oil droplets that may form, creaming or oil separation. The assay was effected by comparison with a known emulsion for parenteral use (Intralipid$^R$ 10%). No change in quality was noticed over the 5 test-day period with respect to the 0.2%, 1% and 3% additions, compared with Intralipid$^R$ 10% lacking such addition. The emulsion containing the 3-percent mixture was found to be fully acceptable over the first 3 days, but slowly deteriorated during day 4 and day 5. The pH of the emulsion was measured after a 5 day storage period. The mean particle size was measured in a Coulter$^R$ nanosizer. The following values were obtained:

| | 5% admix | 3% admix | 1% admix | 0.2% admix | 0% admix |
|---|---|---|---|---|---|
| pH | 6.7 | 6.7 | 6.9 | 7.3 | 7.9 |
| Mean particle size | 244 | 249 | 244 | 241 | 244 |
| | 252 | 246 | 249 | 241 | 236 |
| | 254 | 240 | 244 | 239 | 246 |
| | 252 | 246 | 246 | 239 | 239 |
| | 252 | 236 | 251 | 246 | 244 |
| | | 243 | | | |
| $\bar{X}$ | $\overline{251}$ | $\overline{243}$ | $\overline{247}$ | $\overline{241}$ | $\overline{242}$ |
| S.D. | 3.9 | 4.7 | 3.1 | 2.9 | 4.1 |

The above results indicate that 5-methyl-3-oxo-hexanoic acid ethyl ester is either dissolved into an existing fat emulsion or forms its own emulsion of "normal" micro-type or liposome type. The emulsification or incorporation takes place over a very short time period, with minimum energy consumption. The experiments illustrate a novel method of preparing and supplying a hydrophobic substance under aseptic conditions, without needing to prepare a totally new emulsion.

Example 32

A so-called mother concentrate produced by dissolving 6 g egg-yolk phospholipides in 40 g of 5-methyl-3-oxo-pentanoic acid-ethyl ester at +60° C, while stirring the system. 34 g of 99% ethyl alcohol were then added to the solution, whereafter the solution was cooled to room temperature and dispensed into 20 ml glass flasks. Nitrogen gas was bubbled through the contents of the flasks, and the flasks were subsequently stoppered and fitted with capsules. The flasks were then autoclaved at 120° C and 118 kPa over a period of 20 minutes. This preparation was added to a 20% fat emulsion (Intralipid$^R$) under strict aseptic conditions, such that the concentation of 5-methyl-3-oxo-pentanoic acid ethyl ester was 0.2 and 1 and 3% respectively. The emulsion was shaken for 5 minutes in a shaker and then placed in a cold environment (+5° C). The emulsion was inspected over a period of 5 calender days, wherewith a visual inspection was carried out daily in order to ascertain the occurrence of any oil droplets that may form, creaming or oil separation. The assay was effected by comparison with a known emulsion for parenteral use (Intralipid$^R$ 20%). No change in quality was noticed over the 5 test-day period with respect to the 0.2% and

1% additions, compared with Intralipid^R 20% lacking such addition. The emulsion containing the 3-percent mixture was found to be fully acceptable over the first 4 days, but slowly deteriorated during day 5. The pH of the emulsion was measured after a 5 day storage period. The mean particle size was measured in a Coulter^R nanosizer. The following values were obtained:

| | 3% admix | 1% admix | 0.2% admix | 0% admix |
|---|---|---|---|---|
| pH | 6.7 | 7.0 | 7.4 | 7.7 |
| Mean particle size | 324 | 317 | 317 | 307 |
| | 324 | 317 | 321 | 314 |
| | 328 | 324 | 324 | 317 |
| | 321 | 321 | 324 | 314 |
| | 324 | 317 | 324 | 321 |
| $\bar{X}$ | $\overline{324}$ | $\overline{319}$ | $\overline{322}$ | $\overline{315}$ |
| S.D. | 2.5 | 3.2 | 3.1 | 5.1 |

The above results indicate that 5-methyl-3-oxo-hexanoic acid ethyl ester is either dissolved into an existing fat emulsion or forms its own emulsion of "normal" micro-type or liposome type. The emulsification or incorporation takes place over a very short time period, with minimum energy consumption. The experiments illustrate a novel method of preparing and supplying a hydrophobic substance under aseptic conditions, without needing to prepare a totally new emulsion.

Example 33

For the purpose of investigating the possibility of adding 5-methyl-3-oxy-hexanoic acid ethyl ester to an existing fat emulsion which in addition to triglycerides, water and glycerol also contained egg-yolk phospholipids, 100 ml of fat emulsion 10% respectively 20% (Intralipid^R) were admixed with quantities of pure 5-methyl-3-oxo-hexanoic acid ethyl ester such as to obtain concentrations of 0.2, 1, 3 and 5%. The emulsion was shaken for 5 minutes in a shaking machine and then placed in a cold environment (+5° C). The emulsions were inspected over a period of 5 calender days, wherewith a visual inspection was made daily for the purpose of ascertaining the presence of any oil droplets that may have formed, creaming or oil separation. The assay was effected by comparison with known emulsions intended for parenteral use (Intralipid^R 10% and 20% respectively). The 10% fat emulsion having the 0.2, 1 and 3% concentrations was found to be stable over the whole of the test period, i.e. 5 calender days, whereas the 5% concentration was stable over the first 2 days and then deteriorated. No significant deviations were observed with respect to pH and mean particle size at the end of the 5-day period.

The 20 percent emulsion containing the 0.2 and 1% admixture remained stable over the whole of the test period, i.e. 5 calender days, whereas the 3% mixture remained stable for 1 day. The 5% admixture was unstable. No significant deviations were observed with regard to pH and mean particle size at the end of the 5-day period.

Example 34

For the purpose of investigating the possibility of adding 4-methyl-3-oxy-pentanoic acidethyl ester to an existing fat emulsion which in addition to triglycerides, water and glycerol also contained egg-yolk phospholipids, 100 ml of fat emulsion 10% respectively 20% (Intralipid^R) were admixed with quantities of pure 4-methyl-3-oxo-pentanoic acid ethyl ester such as to obtain concentrations of 0.2, 1, 3 and 5%. The emulsion was shaken for 5 minutes in a shaking machine and then placed in a cold environment (+5° C). The emulsions were inspected over a period of 5 calender days, wherewith a visual inspection was made daily for the purpose of ascertaining the presence of any oil droplets that may have formed, creaming or oil separation. The assay was effected by comparison with known emulsions intended for parenteral use (Intralipid^R 10% and 20% respectively). The 10% fat emulsion having the 0.2, 1, 3 and 5% concentrations was found to be stable over the whole of the test period, i.e. 5 calender days, whereas the 5% concentration was stable over the first 2 days and then deteriorated. No significant deviations were

observed with respect to pH and mean particle size at the end of the 5-day period.

The 20 percent fat emulsion having 0.1 and 1% admixture remained stable over the whole of the test period, i.e. 5 calender days, whereas the 3% admixture remained stable for four days, and the 5% admixture remained stable for 2 days. The pH of the 5% and the 3% emulsions changed significantly, and fell (pH = 5.0 and 5.6 respectively) in comparison with the reference emulsion (pH = 7.9). No other changes were observed.

To summarize Examples 33 and 34, it can be said that 0.2 and 1% admixture can be made in a fat emulsion incor porating an emulsifier which comprises egg-yolk phospholipids dissolved in the oil phase.

## Example 35

Sprague-Dawley male rats weighing 180-190 g were surgically fitted with a central vein catheter protected by a saddle (Roos et al, 1981). Total parenteral nutrition (TPN) was administered in accordance with the following standard formula:

Fat: 8 g/kg body weight and 24 hours
Nitrogen: 1,2 g/kg body weight and 24 hours
Total energy supplied: 1257 kJ/kg body weight and 24 hours
Liquid: 300 ml/kg body weight and 24 hours

The glucose to fat ratio was 70-30 energy percent. The energy percentage obtained from the energy substitutes during the test period corresponded to about 5% NPC. The TPN-mixture was obtained by mixing Vamin$^R$ 14 (a solution of essential and non-essential amino acids for parenteral nutrient administration, retailed by KabiVitrum AB) with no electrolytes, glucose solution 50% and Intralipid$^R$ 20% in suitable proportions and adding adequate quantities of electrolytes, trace minerals and vitamins. Subsequent to infusing the rats continuously for one week (20 hours/day), the animals were divided randomly into three groups, with 6 animals in each group. The animals of one group were administered with the same TPN-formula as that previously mentioned (the reference group). The other groups (the test groups) received 4-methyl-3-oxo-pentanoic acid ethyl ester and 5-methyl-3-oxo-hexanoic acid ethyl ester respectively, in emulsion form. The compounds were given in a dosage of 2 g/kg body weight and 24 hours in a yield corresponding to equivalent glucose energy quantities. In other respects, the TPN-program or formula was the same as that for the reference group. The experiment was then continued for a further 9 days, during which the weights of the animals were recorded daily and urine collected and frozen. At the end of the test period, the rats were killed by heart puncture while under a pentobarbital anesthetic and an autopsy was performed on the rat carcasses with microscopic examination. The body organs were sent to a laboratory for pathological assay. With regard to the reference group, the average growth per day during the whole of the test period was 3.5±0.6 g, whereas the average growth of the rats belonging to the test group that received 5-methyl-3-oxo-hexanoic acid ethyl ester was 2.8±0.2 g, and for the test group receiving 4-methyl-3-oxo-pentanoic acid ethyl ester 3.7±0.8 g. When comparing the growth between the rats of the reference group and the test groups receiving 5-methyl-3-oxo-hexanoic acid ethyl ester, a significant difference in growth in g per day was established. This difference, however, was not significant when the comparison was made of the growth experienced in the last 5 days, which indicates a certain adaptation period. When comparing the test group that received 4-methyl-3-oxo-pentanoic acid-ethyl ester, failed to show any difference from the reference group at any moment of the test period. The weight changes are illustrated in Figure 1. The relative organ weights are shown in Table 3. Wet weight, dry weight and dry solid content are shown in Table 4. No significant deviations between reference groups and test groups could be calculated.

TABLE 3

| | Liver | Spleen | Kidneys | Lungs | Myocardium | Thymus | EDL |
|---|---|---|---|---|---|---|---|
| Reference group | 34.02(2.8) | 2.69(0.3) | 8.18(0.4) | 4.45(0.4) | 4.25(0.3) | 2.55(0.3) | 0.45( |
| Test group B | 36.01(2.5) | 3.40(0.6) | 8.76(0.6) | 4.85(0.1) | 4.04(0.2) | 2.64(0.4) | 0.46( |
| Test group C | 37.41(1.3) | 3.10(0.3) | 9.10(0.4) | 5.26(1.7) | 4.22(0.4) | 2.76(0.4) | 0.48( |

TABLE 4

| Wet weights, dry weights and dry solid contents of some organs. | | | | | | |
|---|---|---|---|---|---|---|
| | Reference group | | Test group B | | Test group C | |
| LIVER: | | | | | | |
| Wet weight (g) | 7.281 | (0.59) | 7.757 | (0.64) | 8.179 | (0.96) |
| Dry content (%) | 33.3 | (0.55) | 34.8 | (1.55) | 33.8 | (1.72) |
| Dry weight (g) | 2.426 | (0.21) | 2.704 | (0.32) | 2.771 | (0.40) |
| KIDNEYS: | | | | | | |
| Wet weight (g) | 1.753 | (0.12) | 1.887 | (0.15) | 1.981 | (0.13) |
| Dry content (%) | 24.9 | (2.65) | 24.5 | (0.99) | 24.7 | (1.62) |
| Dry weight (g) | 0.434 | (0.04) | 0.462 | (0.04) | 0.489 | (0.05) |
| HEART: | | | | | | |
| Wet weight (g) | 0.906 | (0.07) | 0.868 | (0.03) | 0.916 | (0.02) |
| Dry content (%) | 22.2 | (0.72) | 22.5 | (0.95) | 23.2 | (0.41) |
| Dry weight (g) | 0.202 | (0.02) | 0.196 | (0.01) | 0.213 | (0.01) |
| EDL: | | | | | | |
| Wet weight (g) | 0.034 | (0.007) | 0.099 | (0.003) | 0.097 | (0.004) |
| Dry content (%) | 32.2 | (4.54) | 27.3 | (2.11) | 28.4 | (3.07) |
| Dry weight (g) | 0.104 | (0.01) | 0.027 | (0.003) | 0.028 | (0.003) |
| Standard deviations are included in parenthesis. | | | | | | |

In summary, the data indicate that 5-methyl-3-oxo-hexanoic acid ethyl ester and 4-methyl-3-oxo-pentanoic acid ethyl ester can be used as energy substrates at the dosages tested, and have been found to be non-toxic at the same dosages. In the case of 5-methyl-3-oxo-hexanoic acid ethyl ester, however, an inferior weight development was observed during the first days of the test, although this tendency disappeared during the last 5 days of the test period, which indicates a given, belated substrate adaptation.

Example 36

6.75 g glycerol and 6.75 g egg-yolk phospholipids were added to 263.8 g of sterile distilled water. The mixture was heated to +80° C, while stirring the system. The mixture was admixed with an oil comprising 5-methyl-3-oxo-hexanoic acid ethyl ester transesterified with pure soya oil in accordance with Example 25. The mixture was homogenized in an Ultra Turrax$^R$ for 5 minutes. The resultant coarse emulsion, pH = 8.6, was adjusted with 0.8 ml 1 M NaOH and homogenized in a Manton-Gaulin valve homogenizer (model M-15), partly at 15.6 MPa for 3 minutes, partly at 54.9 MPa for 8 minutes, and finally at 15.6 MPa for 3 minutes. Subsequent to homogenization, the temperature was maintained at 50-64° C with the aid of a cooler. The result of the emulsion was stable when steam sterilized in glass flasks (+117° C, 118 kPa for 20 minutes). The emulsion contained transesterified glyceride at an amount corresponding to roughly 55% of the total fat content.

Example 37

6.75 g glycerol, 0.75 g lysine and 6.75 g egg-yolk phospholipids were added to 263.8 g of sterile distilled water. The mixture was heated to +80° C, while stirring the system. The mixture was admixed with an oil comprising 5-methyl-3-oxo-hexanoic acid ethyl ester transesterified with pure soya oil in accordance with Example 25. The mixture was homogenized in an Ultra Turrax$^R$ for 5 minutes. The resultant coarse emulsion, pH = 8.6, was adjusted with 0.8 ml 1 M NaOH and homogenized in a Manton-Gaulin valve homogenizer (model M-15), partly at 15.6 MPa for 3 minutes, partly at 54.9 MPa for 8 minutes, and finally

at 15.6 MPa for 3 minutes. Subsequent to homogenization, the temperature was maintained at 50-64 °C with the aid of a cooler. The result of the emulsion was stable when steam sterilized in glass flasks (+117 °C, 118 kPa for 20 minutes). The emulsion contained transesterified glyceride at an amount corresponding to roughly 55% of the total fat content.

Example 38

6 g of egg-yolk phospholipids were dissolved in 50 g of 5-methyl-3-oxo-hexanoic acid ethyl ester and 4-methyl-3-oxo-pentanoic acid ethyl ester respectively, while applying and stirring the system. When clear solutions had been obtained, 34 g of absolute alcohol (about 99%) were added, corresponding to the least quantity required for the solutions to remain stable in cold conditions without precipitation of phospholipids. These solutions were autoclaved at +117 °C for 20 minutes. Aliquots of these solutions were taken (0.2; 1.0; 3.0 and 5% calculated on ethyl ester) and added respectively to distilled water and to an amino acid solution with a concentration of 7.18% and 9.4 g nitrogen/l (Vamin$^R$ N). Controls were run, in which a pure product of 5-methyl-3-oxo-hexanoic acid ethyl ester and 4-methyl-3-oxo-pen tanoic acidethyl ester respectively were added to distilled water and amino acid solution respectively. All samples were shaken for 10 seconds in a shaking apparatus of the type Whirlimixer$^R$. The samples were then permitted to stand for 5 calender days in cold conditions (+5 °C) and were read-off daily under a daylight lamp, observations being made as to the acceptable quality of the resultant emulsions (visually) or to ascertain the presence or absence of oil droplets on the surface of the emulsions, or separation thereof. The results showed that free pure 5-methyl-3-oxo-hexanoic acid ethyl ester can be mixed in distilled water and in amino acid solution to 0.2%. The relationship concerning phospholipide/ethyl alcohol solutions of the same substance was such as to enable them to be dissolved (in emulsion form) in distilled water to 0.2%; 1.0%; 3.0% and 5.0%, and in amino acid solution to 0.2% and 1.0%.

Pure 4-methyl-3-oxo-pentanoic acid ethyl ester can be mixed with distilled water and amino acid solution to both 0.2 and 1.0%. The relationship concerning phospholipid/ethyl alcohol solution of the same substance was such as to enable them to be dissolved to 0.2%, 1.0%, 3.0% and 5.0% in distilled water and 0.2% and 1.0% in amino acid solution. Accepted samples were those which, in addition to the emulsion quality remaining unchanged over the whole of the 5-day test period, also those samples in which creaming without the formation of oil droplets was observed.

The experiment shows that phospholipids (=emulsifier) are required for emulsions in the referenced system and by the above-tested substances, in order for an acceptable admixture of a desired quantity of the aforedescribed esters to take place.

Example 39

The antiviral effect of 5-methyl-3-oxo-hexanoic acid ethyl ester was tested by investigating, with the aid of known virological methodology, the suppression of replication of VSV (vesicular stomatitis virus) for a number of different cell types in vitro. A549, GMK and fibroblasts. Figure 2 illustrates that replication can be inhibited by using 0.1% 5-methyl-3-oxo-hexanoic acid-ethyl ester in tissue cultures.

## Claims

1. A compound for use as a nutrient substrate, as an antimicrobial and antiviral agent, and/or as an agent for influencing the central nervous system, characterised by the general formula
A - B
where A signifies a carboxylic acid group having a carbon chain which contains 3-10 carbon atoms, preferably 3-6 carbon atoms, and which is preferably branched, and which contains one or more oxo groups bound to one or more carbon atoms in the chain, and B signifies a hydrogen atom, a pharmaceutically acceptable metal atom, an alcohol bound as ester and having 1-5 carbon atoms and with 1-3 hydroxy groups, or a glycerol group bound as ester.

2. A compound according to Claim 1, characterised in that the carboxylic acid group includes one single oxo group which is present in alpha-position or beta-position of the carboxyl group.

3. A compound according to Claim 1 or 2, characterised in that the alcohol bound as ester is a monoalcohol.

4. A compound according to Claim 1 or 2, characterised in that the glycerol group bound as ester is bound to one or more carboxylic acid groups A over its hydroxy groups.

5. A compound according to any one of Claims 1-3, characterised in that the compound comprises one or the compounds
4-methyl-3-oxo-pentanoic acid
5-methyl-3-oxo-hexanoic acid
4-methyl-3-oxo-hexanoic acid
isovaleric acid
pyruvic acid
alpha-keto valinic acid
butyryl acetic acid
or their pharmaceutically acceptable metal salts or ethyl esters.

6. A composition for use as a nutrient substrate, an antimicrobial and antiviral agent, and/or as an agent for influencing the central nervous system, characterised in that the active substance of the composition includes at least one compound according to any one of Claims 1-5.

7. A composition according to Claim 6, characterised in that said composition is formulated for peroral, enteral or parenteral administration, or as an injection or infusion preparation.

8. A composition according to Claim 6 or 7, characterised in that the composition includes the active substance in an amount of 0.01-100 weight/volume percent.

9. A composition according to any one of Claims 6-8, characterised in that it exists in the form of an emulsion of a hydrophobic phase in an isotonic aqueous phase, and in that the hydrophobic phase comprises the actual active substance or a fat or an oil in which the active substance is dissolved or dispersed.

10. A composition according to any one of Claims 6-9, characterised in that the composition also includes at least one basic amino acid.

11. A composition according to any one of Claims 6-10, characterised in that the composition includes additional nutrient substances, such as emulsified fat, essential and non-essential amino acids, and/or carbohydrates.

12. A composition according to any one of Claims 6-11, characterised in that the composition includes additional pharmaceutically active substances, vitamins, trace substances and/or electrolytes.

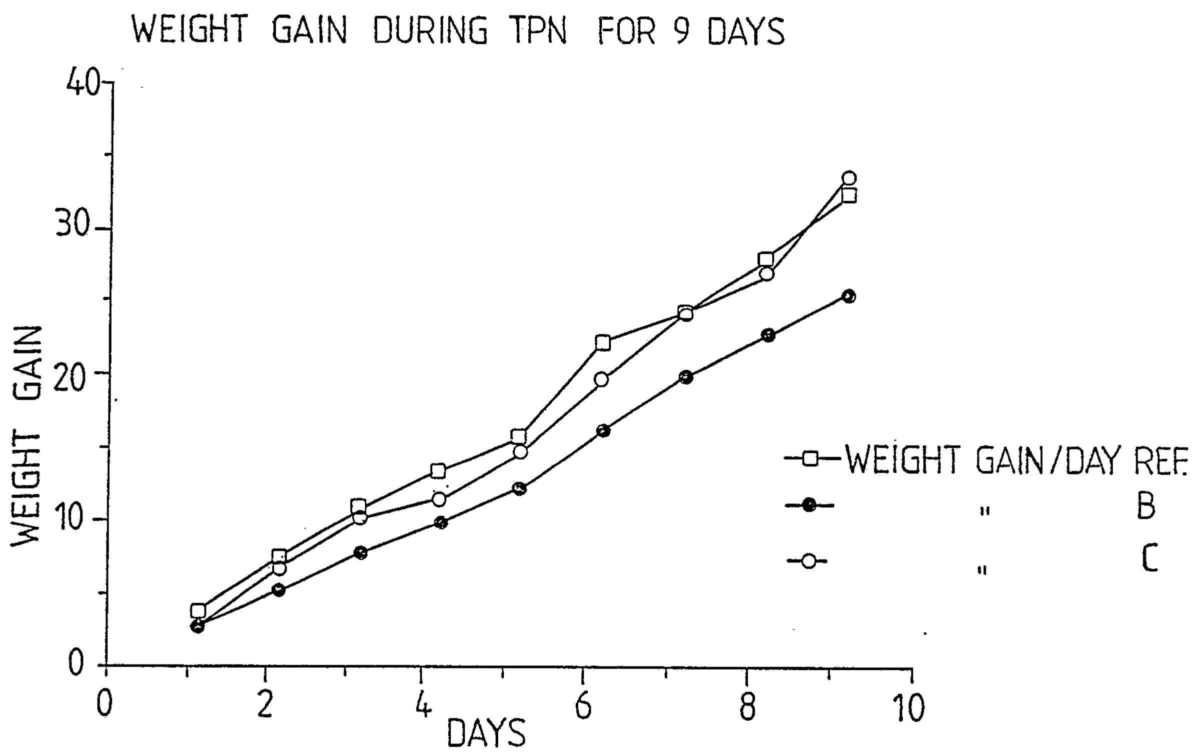
WEIGHT GAIN DURING TPN FOR 9 DAYS
WEIGHT GAIN
40
30
20
10
0
0
2
4
6
8
10
DAYS
WEIGHT GAIN/DAY REF.
" B
" C

FIG.1

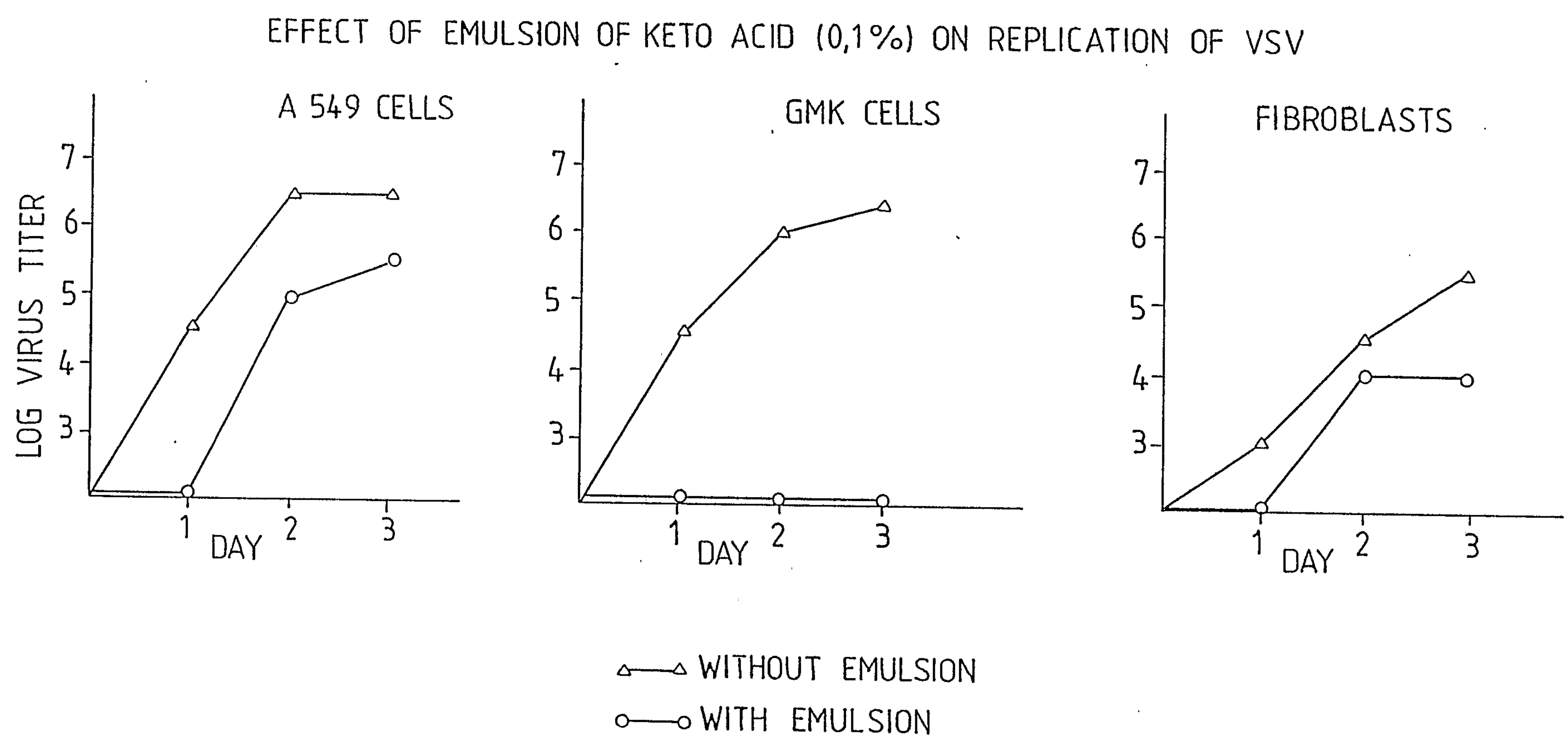
EFFECT OF EMULSION OF KETO ACID (0,1%) ON REPLICATION OF VSV
A 549 CELLS
GMK CELLS
FIBROBLASTS
LOG VIRUS TITER
7
6
5
4
3
1
2
3
DAY
WITHOUT EMULSION
WITH EMULSION

FIG.2

European Patent Office

# EUROPEAN SEARCH REPORT

Application number

EP 89850291.9

## DOCUMENTS CONSIDERED TO BE RELEVANT

| Category | Citation of document with indication, where appropriate, of relevant passages | Relevant to claim | CLASSIFICATION OF THE APPLICATION (Int Cl 4) |
|---|---|---|---|
| X | THE JOURNAL OF CLINICAL INVESTIGATION, vol. 59, March 1977, pages 558-564, BALWANT S. KHATRA ET AL: "Distribution of Branched-Chain α-Keto Acid Dehydrogenases in Primate Tissues". * Page 558 * --- | 1-12 | C 07 C 59/185-59/195<br>C 07 C 69/716-69/72<br>A 61 K 31/19<br>A 61 K 31/22 |
| X | THE JOURNAL OF CLINICAL INVESTIGATION, vol. 50, 1971, pages 90-96, DANIEL RUDMAN: "Capacity of Human Subjects to Utilize Keto Analogues of Valine and Phenylalanine". * The whole document * --- | 1-12 | |
| X | J. NUTRITION, vol. 83, 26 February 1964, pages 85-93, W.G. POND ET AL: "Effects of the α-Hydroxy Analogues of Isoleucine, Lysine, Threonine and Tryptophan and the α-Keto Analogue of Tryptophan and the Level of the Corresponding Amino Acids on Growth of Rats". * The whole document * --- | 1-12 | **TECHNICAL FIELDS SEARCHED (Int. Cl.4)**<br>C 07 C |
| X | JOURNAL OF NUTRITION, vol. 105, 1975, pages 372-378, KYE-WING CHOW ET AL: "Effects of Substitution of Methionine, Leucine, Phenylalanine, or Valine by their α-Hydroxy Analogs in the Diet of Rats[1]". * Page 372 * --- | 1-12 | |
| A | EP, A2, 0 227 545 (SYNTHELABO) 1 July 1987 * Pages 1-3 * --- | 1-12 | |

-/-

The present search report has been drawn up for all claims

| Place of search | Date of completion of the search | Examiner |
|---|---|---|
| STOCKHOLM | 27-11-1989 | JOHANSSON E. |

**CATEGORY OF CITED DOCUMENTS**

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

European Patent Office

# EUROPEAN SEARCH REPORT

Application number

EP 89850291.9

| Category | Citation of document with indication, where appropriate, of relevant passages | Relevant to claim | CLASSIFICATION OF THE APPLICATION (Int. Cl.4) |
|---|---|---|---|
| A | DE, A, 2 335 215 (WALSER MACKENZIE) 31 January 1974 * The whole document * --- | 1-12 | C 07 C 59/185- 59/195<br>C 07 C 69/716- 69/72<br>A 61 K 31/19<br>A 61 K 31/22 |
| A | DE, A, 2 335 216 (WALSER MACKENZIE) 21 November 1974 * The whole document * --- | 1-12 | |
| A | Derwent's Abstract no 67374 D/37, SU 789 504 * Abstract * --- | 1-5 | |
| A | Derwent's Abstract no 11482 B/06 SU 598 869 * Abstract * --- | 1-5 | TECHNICAL FIELDS SEARCHED (Int. Cl.4) |
| A | GB, A, 2 088 354 (SHELL INTERNATIONALE RESEARCH MAATSCHAPPIJ B.V.) 9 June 1982 * The whole document * --- | 1-5 | C 07 C |
| A | THE JOURNAL OF CLINICAL INVESTIGATION, vol. 54, August 1974, pages 271-277, RAJENDER K. CHAWLA ET AL: " Utilisation of α-Keto and α-Hydroxy Analogues of Valine by the Growing Rat". * The whole document * --- | 1-5 | |
| A | EP, A2, 0 232 982 (UNILEVER NV) 19 August 1987 * The whole document * --- | 1-5 | |
| A | DE, A1, 33 34 208 (HENKEL KGAA) 4 April 1985 * The claims * --- | 1-5 | |
| A | GB, A, 903 944 (CHAS. PFIZER & CO. INC.) 22 August 1962 * The whole document * --- | 1-5 | |
| A | DE, A1, 31 44 079 (BASF AG) 19 May 1983 * The whole document * --- -/- | 1-5 | |

European Patent Office

# EUROPEAN SEARCH REPORT

Application number

EP 89850291.9

| DOCUMENTS CONSIDERED TO BE RELEVANT | | | CLASSIFICATION OF THE APPLICATION (Int. Cl.4) |
|---|---|---|---|
| Category | Citation of document with indication, where appropriate, of relevant passages | Relevant to claim | |
| A | Derwent's Abstract no 16935 D/10, SU 745 891 * Abstract * --- | 1-5 | C 07 C 59/185-59/195<br>C 07 C 69/716-69/72 |
| A | Patent Abstract of Japan, Vol. 10, No 13(C-323), abstract of JP 60-169443, publ. 1985-09-02 * Abstract * --- | 1-5 | A 61 K 31/19<br>A 61 K 31/22 |

| TECHNICAL FIELDS SEARCHED (Int. Cl.4) |
|---|
| C 07 C |